# EUROPEAN PATENT APPLICATION

(11) **EP 1 736 099 A2**
(43) Date of publication of application: **27.12.2006**
(21) Application number: 06012220.7
(22) Date of filing: 14.06.2006
(51) Int. Cl.: A61B 5/145, A61B 5/15

(54) **Glucose monitoring kit**

(30) Priority: 23.06.2005 US 159835
(71) Applicant: Forward Industries, Inc., Pompano Beach, FL 33064 (US)
(72) Inventor: Morrison, Andrew, E., Boca Raton FL 33434 (US); Fitzner, Carsten, Los Gatos San Jose CA 95032 (US); Buttermore, Kristopher, W., Fort Lauserdale FL 33301 (US)
(74) Representative: Möhring, Friedrich

(57) **Abstract**

A glucose monitoring kit (10) for storing, carrying and using a blood glucose monitoring system (16), which includes a meter (30), a container (31) for storing test strips and at least one sampler (33), is configured with case (12) having two halves pivotal relative to one another, and
a retention system (14). The retention system (14) includes a plurality of clips (18,20,22) arranged so that the first clip extends along the central region of the case between the two halves and second and third clips coupled to one of the halves. At least one of the first, second and third clips is made from hard polymeric material and configured to removably receive the meter so that the meter can be used by the operator of the monitoring system while being retained in the at least one clip.

## Description

### BACKGROUND OF THE INVENTION

### 1. Field of the Invention

The invention relates to a portable storage assembly for storing, carrying and using health related monitoring systems, and particularly to a case and retention system assembly for blood glucose test kits.

### 2. Discussion of Related Prior Art

A variety of health conditions such as blood pressure and blood glucose levels, to name a few, require constant monitoring. Fairly recently, medical tests could only be administered in clinics. However, recent developments of health-related monitoring systems have provided those in need with a variety of portable and self-administered test kits. Regular administration of the tests, particularly those capable of indicating blood glucose levels, can critically reduce the risk of diabetes complications by up to 60%. Having the monitoring system at hand, thus, has become rather a necessity for people suffering from this condition.

Such a necessity inspired the designers of portable monitoring systems to create a variety of kits typically including a case and retention system, which are adapted to securely hold miscellaneous components of these systems. As any other design, creating a kit for monitoring blood glucose is premised on the fact that the device owner looks for the same qualities in a case as he would in a product carried by this case. Typically, the designers pay a particular attention to functional needs associated with any given case and retention system assembly. A case, in its open state, must provide an easy access to the components of monitoring systems. Hence a particular consideration often needs to be made for making an ergonomically designed arrangement of multiples holders of the retention system.

To meet the ergonomic needs, it is critical to understand the target market and what designs will appeal to this market. Typically, many of the known kits for monitoring blood glucose have multiple holders for various components, which are typically made from stretchable materials. In use, such materials tend to loosen, thereby allowing the retained components of the monitoring system to slip out of their intended locations. Often, stretchable materials can be simply physically damaged. Finally, typically configured as a loop, holders may not be easily manipulated when removing components from or inserting them back into the loop due to the lack of the overall space within the case and inconvenient locations of the holders.

Some of the known kits have been developed with hard plastic cases provided with a retention system. While, esthetically, the hard plastic cases are appealing, their life cycle is limited to a life of a plastic hinge, which, considering the frequent use of the monitoring systems, may not satisfactory.

At least some of the known hard plastic case/retention assemblies are injection molded. Having such a configuration may be rather disadvantageous since an arrangement of individual holders of the retention system often is inconvenient in use of the monitoring system's components. Furthermore, many of the holders may have an unreliable, complicated structure, which often confuses the device operator. Frequently, the case/retention system assembly may be bulky and not easily carried around. All or any of these drawbacks may compel the monitoring system operator to neglect checking his/her blood glucose regularly or even completely abandon this procedure. Needless to say, this can lead to devastating health problems.

A need, therefore, exists for a case provided with an item retention system that is ergonomically configured, durable and esthetically appealing.

A further need exists for a portable assembly that is provided with a structure for securely storing, holding and using a blood glucose monitoring system.

Another need exists for a portable assembly that has a modular retention system including individually molded multiple holders or clips for retaining multiple components of the blood glucose monitoring systems.

Still another need exists for a portable assembly that has a retention system with a single base supporting multiple holders for securely storing, holding and using a group of components of the blood glucose monitoring systems.

### SUMMARY OF THE INVENTION

These needs are satisfied by the inventive portable assembly for carrying, storing and using a health-related monitoring system. While this disclosure is mainly concerned with a blood glucose monitoring system, it will be readily appreciated that other health monitoring systems can be carried, stored and used by the inventive assembly.

In its basic configuration, the inventive assembly includes an openable case and a retention system coupled to the case and configured to removably receive and store a plurality of individual components of a monitoring system for testing blood glucose levels. At least some of the components of the retention system are made from polymeric material and each molded to have a base, which is coupled to the case, and a clip for removably receiving a respective component of the monitoring system.

The inventive retention system can be integrated into any of injection molded, compression molded foam, or sewn cases. As a consequence, such adaptability of the system allows unlimited flexibility with styles and materials selected for manufacturing the case. In contrast to some of the known cases, which are injection molded from single hard material and provided with a plastic hinge, the case of the inventive assembly, preferably, is made from pliable materials. Accordingly, the inventive case is not limited to the lifecycle of the plastic hinge, and, as a consequence, has a long lifecycle, offers an excellent protection for the system's components, and is comfortable to hold, carry and use.

The inventive retention system can be easily modified during a molding process and, thus, can be adapted to a great variety of differently shaped and dimensioned meters and other components of the monitoring system. Made from firm, but flexible material, the inventive retention system reliably protects the retained components from external stresses and, while preventing these components from displacement, allows for their easy insertion, removal and use.

In accordance with one aspect of the invention, the retention system includes a plurality of differently shaped individual clips each shaped and dimensioned to receive a respective one of the monitoring system's components. The clips are configured to have smooth edges and can be easily cleaned, which is particularly important since the use of the monitoring system involves blood.

Organized in an ergonomic manner, the arrangement of the clips provides the system operator with an easy access to the desired component and allows him/her to use at least some of the components without removing them from the clips. This feature is particularly advantageous since a long term study has shown that at least 50% of the system operators including children prefer using the system without removing its components from the case.

In accordance with one modification of the inventive assembly, at least a part of the clips of the retention system are provided with a common base coupled to the case. Each of the clips has an individual configuration specifically adapted to receive and prevent a respective component of the monitoring system from voluntary displacement.

In accordance with a further modification of the inventive assembly, the retentions system is configured with an array of clips each constructed with a respective individual base. Such a modular configuration allows greater adaptability of the retention system to a variety of monitoring systems. Depending on the shape and dimensions of individual components of any given monitoring system, the clips may be arranged so that the individual components of the monitoring system are optimally located for a convenient use by the system operator. In particular, regardless of the configuration of the components, at least some of the clips are so located that the operator can use them without removing these components from the case.

In accordance with still another aspect of the invention, at least some of the clips may be detachably coupled to respective bases and, when detached, can be conveniently suspended to the operator's attire. For example, the body of the meter may be provided with a hook-like assembly that can be carried by the operator in a manner similar to carrying pens, cell phones and the like. Other components of the inventive retention system, notably a sampler, may have the similar hook-like assembly attachable to the cloth of the operator in the same manner. The rational behind the above disclosed configuration is to remind the operator of maybe not enjoyable, but often a life saving procedure that can be conducted in a time-effective manner.

Overall, the case/retention assembly of the present invention is characterized by an easily manufactured, lightweight and simple cost-effective structure that can be adapted to store and retain a variety of health related monitoring systems.

These and other features and aspects of the present invention will be better understood with reference to the following description, figures, and appended claims

### BRIEF DESCRIPTION OF THE DRAWINGS

Figure 1 is a perspective view of an open storage assembly illustrated with a monitoring system and configured in accordance with one embodiment of the invention;
Figure 2 is a perspective view of the inventive assembly of Figure 1 illustrated without the monitoring system;
Figure 3A is a top view of one of modifications of the inventive retention clip;
Figure 3B is a view illustrating attachment of the clip of Figure 3A to the case of the inventive assembly.
Figure 4 is a top view of an open storage assembly illustrated with a monitoring system and configured in accordance with another embodiment of the invention;
Figure 5A is a perspective view of an individual clip of the retention system of the inventive storage assembly, as illustrated in Figure 4;
Figure 5B is an elevational front view of the clip of Figure 5A;
Figure 5C is an elevational side view of the clip of Figure 5A;
Figure 5D is a top view of the clip of Figure 5A;
Figure 6 is a top view of an open storage assembly illustrated with a monitoring system and configured in accordance with a further embodiment of the invention;
Figure 7 is a top view of the clip used in the storage assembly of Figure 6;
Figure 8 is a perspective view of an open storage assembly illustrated with a monitoring system and configured with yet another embodiment of the invention;
Figures 9A and 9B are top-side and bottom-side perspective views of the meter of the monitoring system, respectively, shown with a coupling unit, which engages the storage assembly of Figure 8;
Figure 10 is a top view of an open storage assembly arranged in accordance with a further embodiment of the invention;
Figures 11A, 11B, 11C and 11D are perspective, front, side and top views, respectively, of a clip for retaining the container of the monitoring system;
Figures 12A, 12B, 12C and 12D are perspective, front, side and top views, respectively, of a clip for retaining the sampler of the monitoring system;
Figure 13 is perspective view of an open storage assembly illustrated with a monitoring system and configured in accordance with still another embodiment of the invention;
Figure 14 is a perspective view of the clip of the storage assembly illustrated in Figure 14; and
Figures 15A, 15B, 15C, and 15D are perspective, bottom, side, and top view, respectively, of one of the modifications of the inventive retention clip;

### DETAILED DESCRIPTION

Reference will now be made in detail to several embodiments of the invention that are illustrated in the accompanying drawings. Wherever possible, same or similar reference numerals are used in the drawings and the description to refer to the same or like parts or steps. The drawings are in simplified form and are not to precise scale. For purposes of convenience and clarity only, directional terms, such as top, bottom, left, right, up, down, over, above, below, beneath, rear, and front may be used with respect to the drawings. These and similar directional terms should not be construed to limit the scope of the invention in any manner. The words "connect," "couple," and similar terms with their inflectional morphemes do not necessarily denote direct and immediate connections, but also include connections through mediate elements or devices.

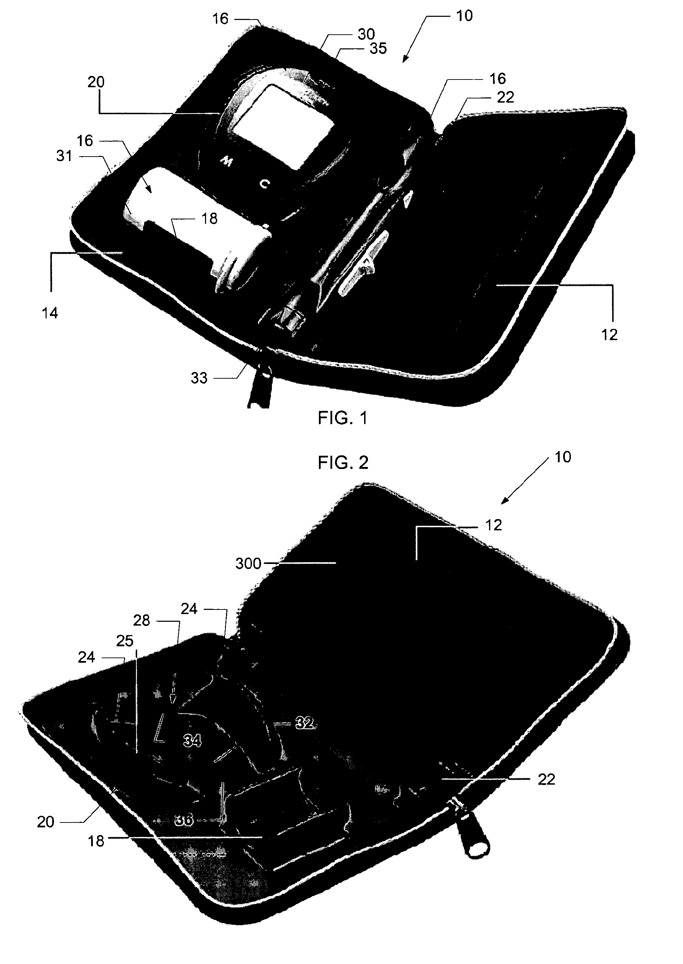

Referring specifically to Figures 1, 2, 3A and 3B, a storage assembly 10 is structured for storing, carrying and using a monitoring system 16 for testing blood glucose levels. The monitoring system 16 may have different components, but usually includes a meter 30, at least one container 31 for storing testing strips and a sampler 33. In addition, monitoring system 16 may include an additional container for storing lancets and a spare sampler. In use, the operator of the monitoring system attaches a lancet to the sampler, pierces his/her skin and collects blood on a test strip, which is removably coupled to the meter after the operator inserts it into a receiving cavity 35. The meter 30, then, is operative to measure and indicate the measured glucose level. Every new test requires a new test strip and, preferably, a new lancet.

The assembly 10 includes a case 12 and a retention system 14 that has a plurality of retainer clips 18, 20 and 22 each configured to removably receive container 31, meter 30 and sampler 33, respectively.

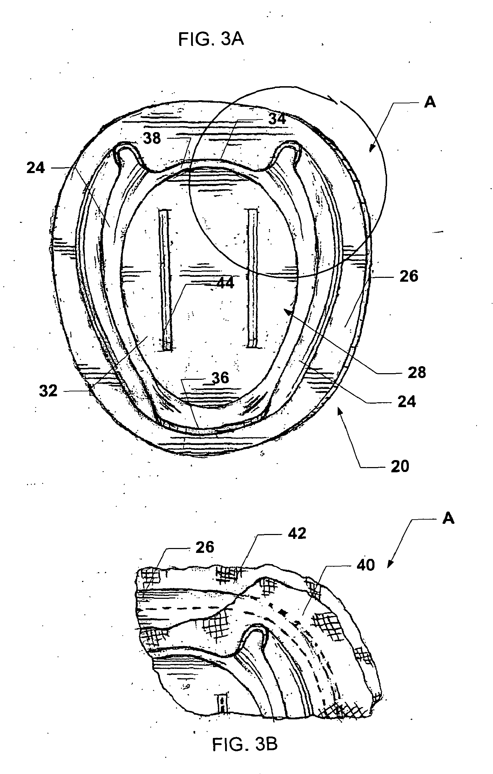

In accordance with one aspect of the invention, retention system 14 is modular. In other words, it may include various configurations of clips whose number, shape and configuration can be adapted for retaining differently structures monitoring systems. As shown in Figures 3A and 3B, for example, clip 20 is molded by any of molding techniques and includes a flat base 26 supporting a nest 28, which is configured to removably receive and retain a meter 30 (Figure 1) of monitoring system 16.

The nest 28 has a bottom 32 and a pair of spaced apart and extending outwardly walls 24, which conform to elongated opposite segments of the periphery of meter 30 once the meter is inserted. To insert meter 30, the monitoring system operator can simply apply an insignificant pushing force to the meter's body whose bottom, while pressing against the top of walls 24, flex these walls away from one another to form the entrance into the space between the walls. As mentioned above, the inner surface of walls 24 is shaped to conform to the opposite sides of the meter, which, as shown in Figure 1, are curved so as to form opposite relatively wide distal and relatively narrow proximal ends 34 and 36 (Figure 2), respectively, of nest 28.

The ends 34 and 36 each are recessed so as to allow the system operator not to remove the meter from the nest while inserting a test strip into the meter through distal end 34. On the other hand, recessed ends 34 and 36 provide walls 24 with the desired flexibility during insertion and removal of the meter. To center meter 30 within nest 28, clip 20 may have a relatively low ledge 38 (Figure 3) bridging end regions 34 and 36. As the meter is guided along walls 24 towards bottom 32, the ledge assists in displacing the meter to its snapping or secured position, in which the walls 24 and ledge 38 of clip 20 surround and lock the meter within nest 28. The secured position of the meter is assured by outer curved edges 25 (Figure 2) extending inwardly from walls 24 and pressing against the top end regions of the front face of the meter.

Attaching each individual clip 18, 20 and 22 to case 12 may be implemented by a variety of techniques depending on material which is selected for manufacturing case 12 and may include, but not limited to, polystyrene, polyethylene and polyurethane. Accordingly, the clip may be glued, bonded or sown to the inner surface of the case. The half of case 12 designed for supporting retention system 14 (Figure 1) may be decorated with one or more layers of decorative material. As illustrated in Figure 3B by an arrow A, base 26 of clip 20 may be placed between layers 40 and 42. In this modification, outer layer 40 is attached to the outer periphery of clip's walls 24 leaving, thus, clip's bottom 32 uncovered. Alternatively, as shown in Figure 2, top layer 40 may be tailored to cover bottom 32.

In the modification shown in Figures 3A and 3B, bottom 32 of nest 28 is formed as a second layer atop base 26 of clip 20. Accordingly, the thickness of the central portion of the clip is greater than its peripheral surface, which, thus, corresponds to the thickness of base 26. To facilitate stitching of the clip to the case, bottom 32 of nest 28 is molded with at least one, but preferably, a plurality of grooves 44 (Figure 3A), which reduce the overall thickness of the central portion of the clip and are easily traversed by a plurality of stitches.

Of course, as will be disclosed below, a great variety of configurations of the inventive clip is envisioned within the scope of the invention. For example, walls 24 of nest 28 may be formed directly on base 26, which, thus, would define the bottom of the nest.

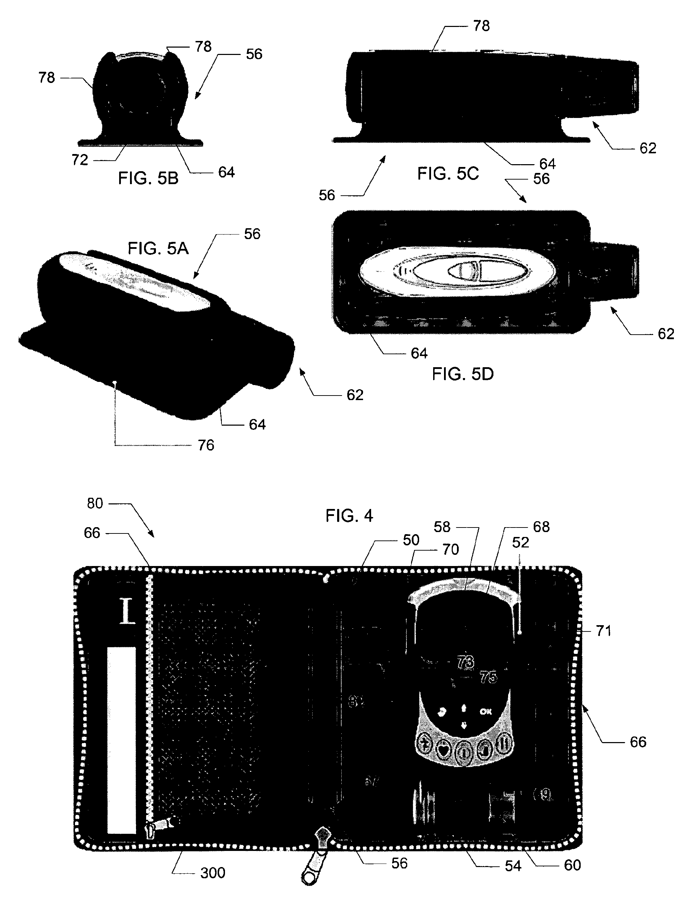

Figure 4 illustrates a further configuration of retention system 50 including retention clips 52, 54 and 56 for storing a meter 58, lancet container 60 and sampler 62 (Figures 5A, 5B, 5C, 5D), respectively. One of the particularities associated with this embodiment includes a different configuration of clip 52 structured to receive meter 58. A peripheral wall of this clip forms a pocket defined by a bottom wall segment 69 and a pair of opposite convex sidewall segments 67 and 71, which have their free distal ends spaced apart so as to form a mouth. During insertion of meter 58, the system operator may either linearly slide meter 58 into the pocket or push the meter inwardly from above into the pocket. Formed from hard polymeric material, the wall segments tend to flex outwards during insertion of the meter and, once the meter is snapped in place, the wall segments urge against the opposing surfaces of the meter preventing its voluntary displacement from the clip. In this embodiment, to conform to the outer periphery of meter 58, the pocket's mouth and bottom wall segment 69 have substantially a uniform width. Furthermore, the configuration of clip 52 allows the operator to easily access and manipulate operating buttons 75 formed on the front face of the meter. The meter may also have a side knob 73 operative, for example, to illuminate the meter's screen 68. Accordingly, sidewall segment 67 of clip 52 is molded with a window 70 allowing the system operator to manipulate knob 73.

Each of the embodiments of the invention, of course, may have clips made from a stretchable material. For example, as illustrated in Figure 4, clip 54, which receives container 60, is formed from stretchable material and has a loop-like structure.

A further particularity of this embodiment of retention system 50 relates to a configuration of clip 56, located along the center region of the case 80 between case halves 66. Better seen in Figures 5A, 5B, 5C and 5D, clip 56 is configured to receive the sampler 62 and, in contrast to the functionally analogous clip shown in Figures 1 and 2 which illustrates two separate nests, has a single elongated body 76 (Figure 5A). Formed with a substantially rectangular base 64, body 76 of clip 56 (Figure 5A) further has a nest defined by a pair of outwardly curved walls 78 (Figure 5B), which run into a generally frustoconical elevated bottom region 72 (Figure 5B) widening towards the base.

To solidify the desired position of the sampler in clip 56 in the closed state of case 80 (Figure 4), retention system 50 is provided with an outwardly open U-shaped seat 82 (Figure 4) located next to one of the walls of clip 56. Upon closing case 80, the bottom of seat 82, which may be covered with material that has a structured surface, encloses the outer portion of sampler 62 and ensures that it will not fall out of the nest.

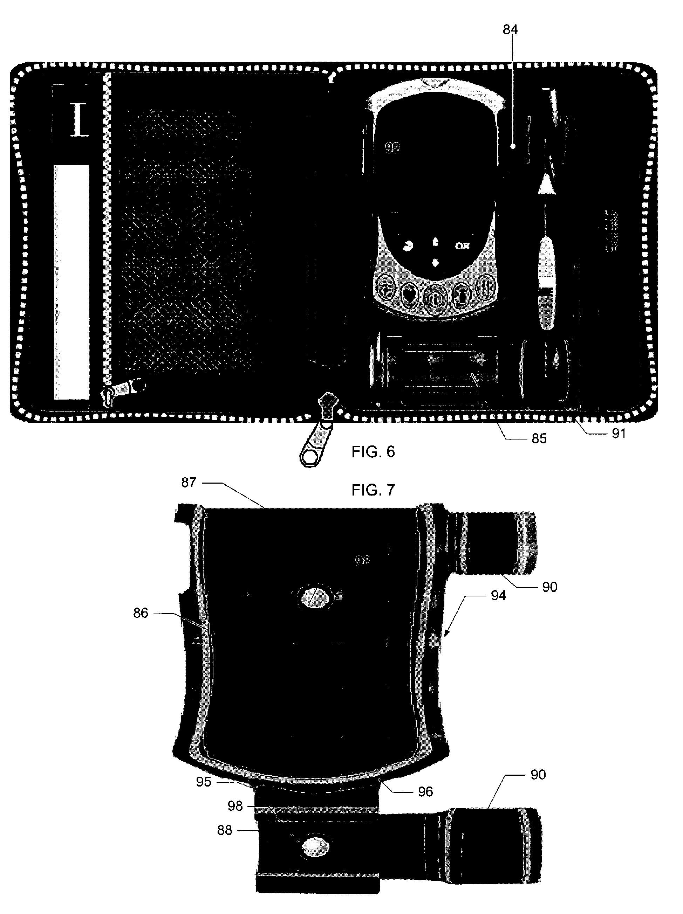

Figures 6 and 7 illustrate a retention system 84 having at least some of the retention clips provided with a common base 94 (Figure 7). Common base 94 supports a clip 86 for a meter, a clip 88 for a container and a two-part clip 90 for a sampler. Accordingly, base 94 has a plurality of differently shaped and sized regions each defining a respective clip.

Clip 86 receiving the meter has a cross-section substantially similar to the one disclosed in reference to the embodiment of Figure 4. Extending from proximal end 96 of clip 86 is a relatively small region of base 95, the free end of which is formed as clip 88 for retaining a lancet or test strip container 85 (Figure 6). Finally, base 94 has two laterally extending regions of clip 90 - one adjacent to a distal end 87 of clip 86 and the other next to clip 88 - which define a bed for the sampler 91 (Figure 6).

In addition, retention system 84 may also have a one-piece clip 92 (Figure 6) located along the central region of the case between the halves. Clip 92 is configured to receive either the same sampler in order to meet individual preferences of the system operator or a spare sampler, because, in general, the sampler may have a tendency of malfunctioning.

As discussed above, the retention system may be attached to the case in various ways. For example, base 94 may be riveted to the case, as illustrated in Figure 7. To accomplish such an attachment, the base has one or more openings 98 which are traversed by fasteners (not shown). The openings 98 may be arranged in a variety of patterns, but preferably, are formed along an axis of symmetry of both clips 86 and 88 configured for receiving the meter and container, respectively.

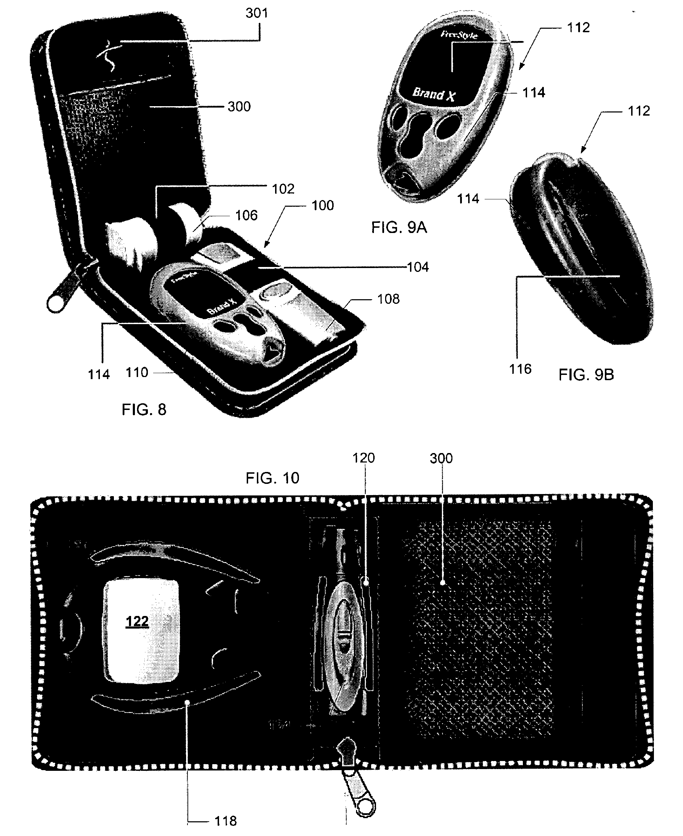

Referring to Figures 8, 9A and 9B illustrating a further modification of retention system 100, which includes two elastic clips 102 and 104 receiving a lancing container 106 and a lancing device or sampler 108, respectively, and a clip 110 for a meter 112. The arrangement of retention system 100 is different from the previously disclosed embodiments in several aspects. For example, clip 102 for receiving the container is formed between the halves of case 301.

Still another difference includes a configuration of clip 110 operative to removably receive and store meter 112. Since the meter is provided with a casing 114 having a plastic lip 116 (Figures 9A and 9B) that allows the operator to carry the meter separately from the case, clip 110 may be provided with a respective plastic loop (not shown) engageable by lip 116. Alternatively, of course, clip 110 may have the same configuration as, for example, any of the clips specifically configured to receive the meter as is disclosed previously.

A further configuration of the retention system, as illustrated in Figure 10, includes only two clips 118 and 120 receiving a meter 122 and a sampler 124, respectively. The clip 120 is located between the halves of the case and may have any of the previously disclosed clip configurations adapted to receive the sampler. Similarly, clip 118 may have any of the previously disclosed structures for retaining the meter.

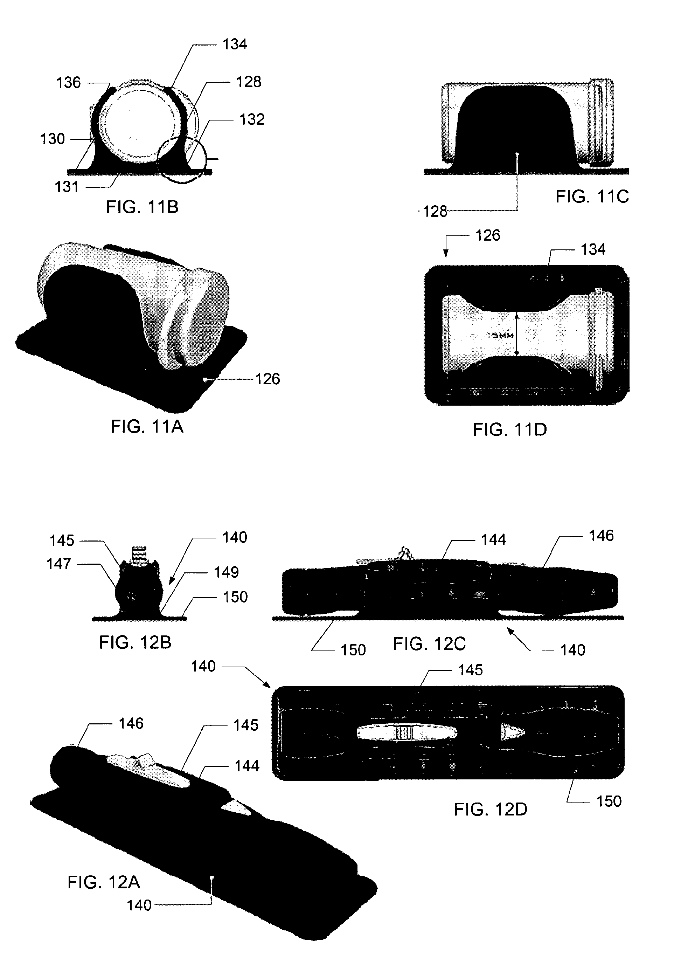

Figures 11 A, 11B, 11C and 11D illustrate a clip 126 configured to retain a container for lancets or test strips. As the clips disclosed above, clip 126 has a base 130 and a pair of sidewalls 128 extending outwards from the base. Each of sidewalls 128 has a concave outer portion 136 provided with an outer edge 134 (Figure 11 D) and a convex inner portion 132. The operator may use the container both in its retained or removed position. Since the container has a relatively large peripheral surface, as compared to the sampler, its displacement into or from the clip may generate substantial flexing forces upon sidewalls of the body of clip 126. To withstand these forces, inner wall portions 132 of the walls 128 are spaced from one another so as to define a wide transitional bottom region 131, and each inner portion terminates substantially in the same plane as the apex of a respective one of outer wall portions 136.

The outer edges 134 of sidewalls 128 are configured to conform to the circular periphery of the container. To provide the operator with a comfortable grip, preferably, edges 134 cover approximately between 1/3 - ½ of the top segment of the container in the inserted position thereof, as can be seen in Figure 11B, 11C and 11D.

A clip 140, as shown in Figures 12A, 12B, 12C and 12D, is configured to retain a sampler 146, which can be inserted in response to a pushing force sufficient to flex the clip's sidewalls from one another so as to clear the entrance into the nest of the clip. The walls 144 are provided along the central, relatively wide portion of sampler 146 and each have an upper substantially rectilinear portion 145 (Figure 12B), an intermediary outwardly curved portion 147 and an inwardly curved bottom portion 149 which runs into a base 150. The distance between the upper portions 145 of the sidewalls is substantially the same as the width between bottom portions 149 and somewhat smaller than the distance between the apexes of intermediary portions 147. Such a configuration allows the walls to reliably hold the inserted sampler while remaining flexible enough to yield to an insignificant pulling force generated by the system operator during removal of the sampler from the clip.

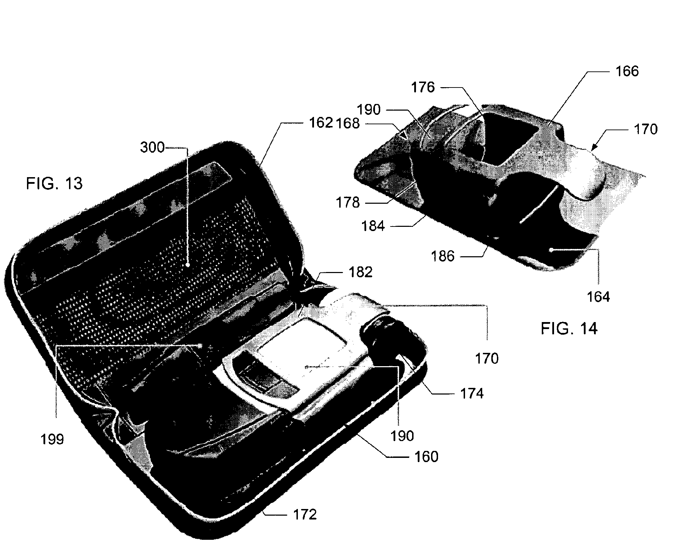

Figures 13 and 14 illustrate a further configuration of a retainer system 160 arranged in a space-effective manner so as to fit a relatively small case 162. The retention system 160 includes a base 164 supporting a single clip body 166, which has two clips 168 and 170 for receiving a meter 172 and a container 174, respectively. In addition, the retention system also has a loop 199 located along the central region of case 162 and structured to receive a sampler.

Clip 168 is structured with a pocket 176 provided with an outer wall 182, which is spaced from base 164, and defined between sidewalls 184 and a distal wall 186. The proximal end 178 of pocket 176 is open so as to allow meter 172 to slide into and out of the pocket in response to a linear force applied by the system operator or to use the meter with a test strip without actually removing the meter from the pocket. The outer wall 182 has a plurality of openings 190 that provides the operator with an easy access to the operating buttons and clear vision of the meter's screen.

Extending from the distal end of top wall 182 is clip 170 that has an outwardly curved arm provided with an inner surface, which conforms to the outer periphery of container 174. A distance between the inner surface of clip 170 and base 164 is selected to be slightly smaller than the diameter of the container. Accordingly, when the system operator is inserting the container, clip 170 initially slightly flexes upwards and, upon insertion of the container, presses the container downwards against base 164 so as to prevent the container from voluntary displacement.

The case of the inventive storage assembly typically has two halves, as has been pointed out throughout the disclosure. Typically, the retainer system is located within one of the halves with the exception of those clips that can be placed between the halves. Placing a retainer along the spine of the container provides additional structural rigidity to the container. The other half may be provided with a single or multiple pockets 300 (Figures 2, 4, 8, 10, and 13), which are typically made from mesh and configured to removably receive various literature associated with the use of the monitoring system. Closing the case and pockets is typically provided by a zipper, although, of course, other means, such as Velcro strips, pushbuttons and the like, can be implemented as well.

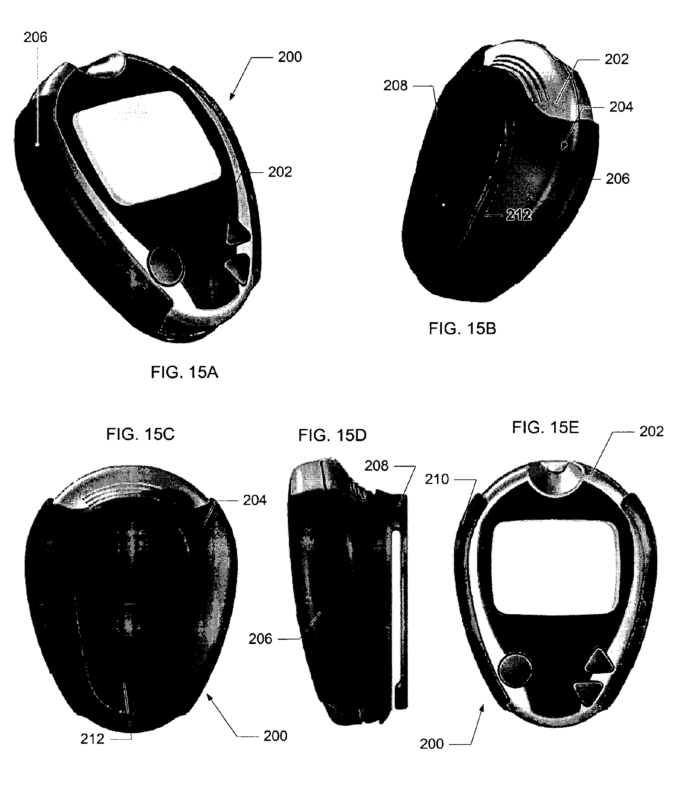

Referring to Figures 15A, 15B, 15C, 15D and 15E, another modification of a clip 200 for receiving a meter 202 is shown. Based on the same concept, as is disclosed in reference to Figures 9A and 9B, clip 200 can be used separately from the rest of the inventive storage assembly. Alternatively, the clip can be integrated into any of the disclosed modifications of the retention system.

The clip 200 includes a tray-shaped body 204 provided with a bottom 212, which is slightly sunk inwards and provided with a peripheral wall 206. Recessed at opposite proximal and distal ends, the wall also has a top rounded edge 210 which abuts top peripheral regions of the meter after the latter has been inserted into the clip. Made from molded TPR or rubber, like all of the disclosed clips, body 204 is sufficiently flexible to have its wall 206 yield to an external pushing force applied by the system operator to the meter. As is disclosed above, after the meter has been inserted, the wall resiliently presses against the opposing surfaces of the meter and prevents the latter from voluntary displacement.

The clip 200 may be integrated into the case of the inventive assembly (not shown) by having a clip pin 208 engaged with a respective hook portion that can be detachably coupled either directly to the case or to the base (not shown), which, in turn, is attached to the case. When, the system operator wishes to carry the meter without the case, he/she may either remove the meter from clip 200 or leave the meter within the clip and detach the latter from the case. Using clip pin 208, the operator can suspend the clip with the meter to the desired part of the operator's attire.

This document describes the inventive storage assembly whose specific disclosed embodiments do not limit the general principles underlying the invention. In particular, the invention is not limited to any particular materials, or arrangements of the retention system, since any specific arrangement of the system's clips depends upon the configuration of the monitoring system and the number of its components. Some of the molded clips can be replaced with loops of stretchable material, and conversely. The specific features described herein may be used in some embodiments, but not in others, without departure from the spirit and scope of the invention as set forth. Many additional modifications are intended in the foregoing disclosure, and it will be appreciated by those of ordinary skill in the art that in some instances some features of the invention will be employed in the absence of a corresponding use of other features. The illustrative examples therefore do not define the metes and bounds of the invention and are subject to further developments and modifications of the illustrated embodiments, as defined in the appended claims.

## Claims

1. A portable storage assembly for a health related multi-component monitoring system, the portable storage assembly comprising:
a case;
a retention system mounted to an interior of the case and having a plurality of clips, the plurality of clips each being configured to removably receive a respective one of multiple components of the monitoring system, at least one of the plurality of clips being configured so that a respective one of the received components can be used by an operator of the monitoring system while being retained in the at least one clip.

2. The portable storage system of claim 1, wherein the at least one clip includes a base coupled to the interior of the case and a peripheral wall extending outwards from the base, the peripheral wall having an outer edge defining an opening, the peripheral wall being resiliently deflectable so as to increase the opening during insertion and removal of the respective component of the monitoring system into and out of the at least one clip, respectively.

3. The portable storage system of claim 2, wherein the peripheral wall of the at least one clip has at least two oppositely spaced peripheral sidewall segments conforming to and pressing against an outer periphery of the respective component of the monitoring system upon insertion thereof into the at least one clip by the operator so as to prevent voluntary displacement of the respective component in the case.

4. The portable storage system of claim 2, wherein the outer edge extends along a border region of an outer face of and presses the respective component inwards against the base of the at least one clip upon insertion of the respective component into the at least one clip.

5. The portable storage system of claim 2, wherein the base and peripheral wall of the at least one clip are made from hard polymeric thermoplastic material or rubber as a unitary body.

6. The portable storage system of claim 3, wherein the peripheral wall has a distal and proximal end segments bridging the side wall segments, at least one of the distal and proximal end segments being configured to allow the operator of the monitoring system to manipulate the respective component without detachment thereof from the side segments of the at least one clip.

7. The portable storage system of claim 6, wherein the monitoring system is configured to monitor blood glucose levels and includes at least a sampler operative to draw blood of the operator of the monitoring system, a container for storing testing strips each configured to collect the drawn blood and a meter removably coupleable to each of the testing strips and operative to measure and indicate a measured blood glucose level, the case having two halves coupleable to one another to enclose the monitoring system.

8. The portable storage system of claim 7, wherein the respective component is the meter configured to selectively receive the testing strips through the at least one recessed distal or proximal segment of the peripheral wall of the at least one clip of the retention system, whereas the meter is operative to measure and indicate the measure blood glucose level without being removed from the case.

9. The portable storage system of claim 7, wherein the at least one of proximal and distal segments is the proximal segment configured to facilitate displacement of the respective component into and out of the at least one clip between the side wall segments in response to an external force applied by the operator of the monitoring system to the respective component.

10. The portable storage system of claim 7, wherein the at least one clip is configured to receive the sampler and includes a single elongated body extending between opposite ends of the sampler or a pair of spaced apart body parts each receiving a respective one of the opposite ends of the sampler.

11. The portable storage system of claim 10, wherein the side wall segments of the peripheral wall of the at least one clip each have a respective outer portion, convex inner portion spaced from the outer portion and a concave middle portion bridging the outer and inner portions, the middle concave portion of the sides segments of the peripheral wall extending laterally beyond the convex inner and outer portions of the peripheral wall so as to conform to an outer periphery of the respective component.

12. The portable storage system of claim 7, wherein the sidewall segments of the peripheral wall of the at least one clip are configured to receive the container therebetween and each have a respective inner surface conforming to an outer periphery of the container and terminating so that an outer edge of each sidewall segment is juxtaposed with and presses inwards against an outer periphery of the retained container, whereas the sidewall segments define an enlarged grip area for the operator of the monitoring system during displacement of the container in and from the at least one clip.

13. The portable storage system of claim 7, wherein the halves of the case are adjoined to one another along a central region of the case and operative to pivot about the central region between a closed and open position of the case, the at least one clip being located in the central region and configured to removably receive the sampler or container of the monitoring system.

14. The portable storage system of claim 2, wherein the retention system has at least one second clip for receiving a second component of the monitoring system, the at least one second clip having a respective base and peripheral wall coupled to and extending outwards from the respective base, the bases of the at least one and second clips being spaced apart.

15. The portable storage system of claim 2, wherein the retention system has a second clip configured to removably receive a second component, the second clip having a respective base and a respective peripheral wall coupled to and extending outwards from the base, the bases of the at least one and second clips defining a one-piece base coupled to the interior of the case and having the peripheral walls of the at least one and second clips spaced apart on the one one-piece base.

16. The portable storage system of claim 15, wherein the one-piece base is configured with:
a first region supporting the peripheral wall of the at least one clip, the peripheral wall of the at least one component having a pair of sidewall segments bridged by a proximal bottom segment so that the sidewall and bottom segments of the at least one clip define a pocket provided with an open distal end and configured to removably receive the respective component of the monitoring system so as to provide access thereto through the open distal end; and
at least one second region running outwards from the first region of the one-piece base and supporting the peripheral wall of the second clip, the peripheral wall of the second clip being recessed so as to define a generally U-shaped nest for removably receiving the second component of the monitoring system.

17. The portable storage system of claim 16, wherein the one-piece base has a third region extending laterally from the first and second regions and configured to removably receive a third component of the monitoring system, the third region of the one-piece base supporting a third clip and being configured as a single-part body or a multi-potion body.

18. The portable storage system of claim 2, wherein the at least one clip further has an outer wall bridging the peripheral wall so that the outer wall, peripheral wall and base define a pocket, at least one of opposite distal and proximal ends of the at least one clip being open so as to receive the respective component of the monitoring system.

19. The portable storage system of claim 18, wherein the outer wall has an arm extending beyond a distal end of the pocket and having a curved inner surface, the curved inner surface being spaced from the base at a distance selected so that the arm flexes outwards during insertion of a second component of the monitoring system and presses thereupon upon insertion of the second component to prevent displacement of the second component relative to the at least one clip.

20. The portable storage system of claim 2, wherein the respective component has a shield provided with a hook configured to attach to a cloth of the operator of monitoring system upon removal of the respective component from the at least one clip.

21. The portable storage system of claim 20, wherein the respective component is a meter operative to measure and indicate blood glucose levels shield has an outer face provided with a plurality of windows configured to provide the operator of the monitoring system with an access to a plurality of operating buttons and a screen of the meter.

22. The portable storage system of claim 6, wherein the respective component is a meter operative to measure and indicate levels of blood glucose and having a plurality of operating buttons, one of the side segments of the peripheral wall of the at least one clip having a throughgoing hole configured to be traversed by one of the operating buttons so as to be accessible by the operator of the monitoring system when the respective component is engaged with the at least one clip.

23. The portable storage system of claim 7, wherein the case is made from pliable material, one of the halves of the case being provided with at least one closable pocket made from mesh and configured to receive literature regarding the monitoring system.

24. The portable storage system of claim 7, wherein the case has a bottom covered with a plurality of fabric layers arranged so that the base of the at least one clip is sandwiched between and coupled to the plurality of fabric layers.

25. A portable storage system for storing, carrying using a blood glucose monitoring system including a meter, a container for storing test strips and at least one sampler, the portable storage system comprising:
a case having two halves pivotal relative to one another between a close and open position of the case; and
a retention system including a plurality of clips arranged so that a first clip of the plurality of clips extends along a central region of the case between the two halves and second and third clips are coupled to one of the halves, at least one of the first, second and third clips being made from hard polymeric material and configured so that a respective one of the received components can be used by an operator of the monitoring system while being retained in the at least one clip.

26. The portable storage system of claim 25, wherein the retention system is provided with a base common to at least two of the first, second and third clips.

27. The portable storage system of claim 25, wherein the retention system is provided with a first, second and third bases spaced from one another and supporting the first, second and third clips, respectively.

28. The portable storage system of claim 25, wherein the at least one clip is configured to removably receive the meter and has a base coupled to an interior of the case and a peripheral wall extending outwards from the base, the peripheral wall having an outer edge defining an opening, the peripheral wall being resiliently deflectable so as to increase the opening during insertion and removal of the meter into and out of the at least one clip, respectively.

29. The portable storage system of claim 28, wherein the peripheral wall of the at least one clip has at least two oppositely spaced peripheral sidewall segments conforming to and pressing against an outer periphery of the meter upon insertion thereof into the at least one clip by the operator so as to prevent voluntary displacement of the respective component in the case.

30. The portable storage system of claim 29, wherein the outer edge extends along a border region of an outer face of and presses the meter inwards against the base of the at least one clip upon insertion of the meter into the at least one clip.
